(19) Europäisches Patentamt
European Patent Office
Office européen des brevets

(11) EP 3 383 357 B1

(12) **EUROPEAN PATENT SPECIFICATION**

(45) Date of publication and mention of the grant of the patent:
**21.07.2021 Bulletin 2021/29**

(21) Application number: **15821226.6**

(22) Date of filing: **17.12.2015**

(51) Int Cl.:
***A61K 8/38** (2006.01)* ***A61K 8/46** (2006.01)*
***A61K 8/49** (2006.01)* ***A61Q 11/02** (2006.01)*
***A61K 8/22** (2006.01)*

(86) International application number:
**PCT/US2015/066292**

(87) International publication number:
**WO 2017/105456 (22.06.2017 Gazette 2017/25)**

(54) **HYDROGEN PEROXIDE BOOSTER SYSTEM FOR ENHANCED TEETH WHITENING**

WASSERSTOFFPEROXIDVERSTÄRKERSYSTEM ZUR VERBESSERTEN ZAHNBLEICHUNG

SYSTÈME D'INTENSIFICATION DE PEROXYDE D'HYDROGÈNE POUR BLANCHIMENT DES DENTS AMÉLIORÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.10.2018 Bulletin 2018/41**

(73) Proprietor: **Colgate-Palmolive Company**
**New York, NY 10022 (US)**

(72) Inventors:
- **DOGO-ISONAGIE, Cajetan**
  **Highland Park**
  **New Jersey 08904 (US)**
- **XU, Guofeng**
  **Plainsboro**
  **New Jersey 08536 (US)**

(74) Representative: **Sonnenhauser, Thomas Martin**
**Wuesthoff & Wuesthoff**
**Patentanwälte PartG mbB**
**Schweigerstraße 2**
**81541 München (DE)**

(56) References cited:
**US-A1- 2005 281 773** **US-A1- 2006 171 906**
**US-A1- 2009 311 198** **US-A1- 2015 265 511**

- **Guan Wang: "Synthesis of Bleach Activators with Varying Cationic Groups", , 1 January 2012 (2012-01-01), XP055266673, Retrieved from the Internet: URL:http://repository.lib.ncsu.edu/ir/bitstream/1840.16/7695/1/etd.pdf [retrieved on 2016-04-19]**
- **None**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 383 357 B1

## Description

### BACKGROUND

Disclosed herein are novel systems and methods for forming novel tooth whitening compositions that include a bleach activator for enhanced whitening,

In a mammal, a tooth is comprised of an inner dentin layer and an outer hard enamel layer that is the protective layer of the tooth. The enamel layer of a tooth is naturally an opaque white or slightly off-white color. It is the enamel layer that can become stained or discolored, although the dentin layer can also be stained which has been shown to happen naturally over time. The enamel layer of a tooth is composed of hydroxyapatite mineral crystals that create a somewhat porous surface. These hydroxyapatite crystals form microscopic hexagonal rods or prisms that make up the enamel surface. As a result, the surface of the enamel layer presents microscopic spaces or pores between the prisms. It is believed that this porous nature of the enamel layer is what allows staining agents and discoloring substances to permeate the enamel and discolor the tooth. These remaining substances can occupy the microscopic spaces and eventually alter the color of the tooth.

Many substances that a person confronts or comes in contact with on a daily basis can stain or reduce the whiteness of one's teeth. In particular, the foods, tobacco products, and fluids that one consumes tend to stain one's teeth. These products or substances tend to accumulate on the enamel layer of the tooth and form a pellicle film over the teeth.

These staining and discoloring substances can then permeate the enamel layer. This problem occurs gradually over many years, but imparts a noticeable discoloration of the enamel of one's teeth. A product or substance that would whiten the discolored teeth would be advantageous. It is also essential that a tooth whitening product that is to be used at home or in private by the consumer be safe and easy to use and be stable and retain its whitening efficacy during its storage on retail store shelves as well as over the period of use by the consumer.

Products and substances that are presently available to whiten teeth, known as tooth whitening compositions, may include a variety of different ingredients formulated into a liquid, paste or gel carrier. The primary active ingredient in such products and substances that provides whitening action, known as the whitening agent, is a peroxide which whitens teeth by oxidizing the organic matrix of teeth.

There is a need in the art to enhance whitening action over that of conventional tooth whiteners. Accordingly, there is a need in the art for tooth whitening systems for storing and providing such improved tooth whitening compositions.

### BRIEF SUMMARY

In an embodiment there is a tooth whitening system, including at least one whitening agent disposed in a first storage chamber, and at least one bleach activator disposed in a second storage chamber.

In another embodiment, there is a method for whitening a surface of a tooth. The method includes combining at least one whitening agent and at least one bleach activator, contacting the surface of a tooth with the tooth whitening composition for a duration of time sufficient to effect whitening of the surface of the tooth.

In another embodiment, there is a method for making a tooth whitening system. The method includes at least partially filling a first storage chamber with at least one whitening agent and at least partially filling a second storage chamber with at least at least one bleach activator.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

The present invention will become more fully understood from the detailed description and the accompanying drawings, wherein:

**FIG. 1** is a cross-sectional view of a product dispenser of an embodiment.

**FIG. 2** is a perspective drawing illustrating dispensing a composition from the dispenser of FIG. 1 onto a mouth-tray.

**FIG. 3** is a graph showing whitening effect between a conventional tooth whitening composition that does not include a bleach activator and a tooth whitening composition of an embodiment that does include a bleach activator.

**FIG. 4** is a graph that shows relative increased whitening effect at 7 treatments and 14 treatments between a commercial tooth-whitening formulation and a tooth-whitening formulation of an embodiment.

**FIG. 5** is a graph showing stability of whitening agent ($H_2O_2$) wt% between 0, 1 and 2 weeks for a conventional

tooth-whitening formulation and that of a formulation of the embodiments both of which were aged at 60°C for 2 weeks. Results show that significant amount of $H_2O_2$ remains after aging study.

## DETAILED DESCRIPTION

The scope of the present invention is defined in the appended claims. The following description of the preferred embodiment(s) is merely exemplary in nature and is in no way intended to limit the invention, its application, or uses.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

Disclosed herein are tooth whitening compositions comprising at least one whitening agent, and at least one bleach activator for enhanced whitening comprising an activated ester. The inventors have surprisingly discovered that a bleach activator provides enhanced whitening when combined with a whitening agent. In one embodiment, the bleach activator that provides enhanced whitening may comprise an activated ester.

Also disclosed herein is a multi-component, such as a two-component, tooth whitening system that may include a whitening agent as a first component and a bleach activator as a second component. The first and second components may be kept separate, such as disposed in separate storage chambers, for example, disposed in separate chambers of a multi-component product dispenser. Upon mixing the first component and the second component, a tooth-whitening composition may be formed. In an embodiment, the tooth whitening system may further include a mixing chamber, connected to both the first storage chamber and the second storage chamber, that receives and sufficiently mixes contents from the first storage chamber and the second storage chamber. Such a tooth whitening composition may provide superior and faster whitening compared to a tooth composition that includes the whitening agent but that does not include the bleach activator. The tooth whitening compositions disclosed herein may further comprise other additional ingredients that include those known to one of skill in the art, including one or more of the following components: fluoride ion sources, surfactants, flavoring agents, sweeteners, desensitizing agents, antimicrobial agents, anti-caries agents, anti-calculus agents, tartar control agents, anti-inflammatory agents, buffering agents, vitamins, pigments and coloring agents, preservatives, and enzymes, as will be discussed in greater detail below.

In some embodiments, the tooth whitening compositions, first components, second components, bleach activator and whitening agent disclosed herein are viscous liquids, such as gels, thereby enabling the product to be applied to the tooth surface, such as with a soft applicator pen or brush, or a mouth-tray. In an embodiment, at least one of the whitening agent and the bleach activator may be combined with an orally acceptable vehicle, and then further combined with each other to form a tooth whitening composition which may include a dentifrice. Such dentifrices may include a dental tablet, toothpaste (dental cream), tooth powders, or gel, or any other form known to one of skill in the art. As used herein, an "orally acceptable vehicle" refers to a material or combination of materials that are safe for use in the compositions of the present disclosure, commensurate with a reasonable benefit/risk ratio, with which the whitening agent, and other desired active ingredients may be associated while retaining significant efficacy.

In some embodiments, the tooth whitening composition disclosed herein is substantially anhydrous, meaning that substantially no water is added. The tooth whitening composition of embodiments may comprise trace levels of water from ingredients or from product manufacture; however, such trace levels are insubstantial and do not interfere with the stability of the tooth whitening composition. In an embodiment, the vehicle is a low water content orally acceptable vehicle and may include any known ingredients or additives. For example, the vehicle may include liquid mixtures of glycerin, and sorbitol.

The tooth whitening composition is formed by mixing a whitening agent and a bleach activator. The whitening agent may be a first component and the bleach activator may be a second component of a multi-component tooth whitening system, such as the multi-component tooth whitening system described herein. In the method, the whitening agent and the bleach activator are mixed, for example, by combining the whitening agent and the bleach activator provided from their separate storage chambers, to form the tooth whitening composition. In an example, the whitening agent and the bleach activator are mixed in or on an applicator such as a mouth-tray.

As further detailed below, the whitening agent and the bleach activator are stored separately until time of use, such as until mixing them together to form the tooth whitening composition. The separation of the two components, that is, the whitening agent and the bleach activator disposed in separate storage chambers, until time of use, such as until they are mixed to forth the tooth whitening composition, provides for increased stability of each of the whitening agent peroxide and bleach activator.

The tooth whitening compositions described herein includes a first component, such as at least one whitening agent, and a second component, such as at least one bleach activator. The bleach activator may comprise an activated

ester. Each of the first and the second components may further comprise other additional ingredients that include those known to one of skill in the art, including one or more of the following components: fluoride ion sources, surfactants, flavoring agents, sweeteners, desensitizing agents, antimicrobial agents, anti-caries agents, anti-calculus agents, tartar control agents, anti-inflammatory agents, buffering agents, vitamins, pigments and coloring agents, preservatives, and enzymes, as will be discussed in greater detail below. The buffering agents may be added with the whitening agent and/or with the bleach activator in their respective separate storage chambers, or via an additional storage chamber, such that mixing the first component and the second component forms a tooth whitening composition that comprises an appropriate pH, such as an alkaline pH, for example, for maximum reaction between the whitening agent and the bleach activator. In an embodiment, at least one of a buffering agent may be added to the whitening agent and/or the bleach activator in a first one of a storage chamber and a second one of a storage chamber, respectively, and the at least one of a buffering agent included with whitening agent in the first storage chamber may be different than the at least one of a buffering agent added with the bleach activator in the second storage chamber. Any one or more of these additional ingredients may be included in the first component that includes the tooth whitening agent and/or in the second component that includes the bleach activator. The same or different one or more of these additional ingredients may be included in the first component than in the second component.

**FIG. 1** is a cross-sectional view of a product dispenser 100, where the product may be an oral care product. The dispenser may include two storage chambers, 101 and 103 (holding contents A and contents B), a mixing chamber 105 that receives and sufficiently mixes contents from the two storage chambers, and a dispensing nozzle 106 in fluidic communication with the mixing chamber. In other embodiments, more than two storage chambers 101 and 103 may be included. In an example, contents A may include the whitening agent and may include at least one of a buffering agent. In an example, contents B may include the bleach activator and may include at least one of a buffering agent. The at least one of the buffering agent in contents A may be the same or different than at least one of the buffering agent in contents B.

In an example, the contents comprise first contents disposed in a first one of the at least two storage chambers, second contents disposed in a second one of the at least two storage chambers. At least one of the first and the second contents may comprise an oral care product. In an example, contents from each of the storage chamber are mixed in the mixing chamber. The dispensing nozzle receives the mixed contents from the mixing chamber and the mixed contents may include an oral care product.

A seal 102 and a seal 104 keep contents stored in the two storage chambers separated during storage. In the embodiment shown, the seal 102 may be disposed between the mixing chamber 105 and the storage chamber 101, on the end of the inlet 109, and the seal 104 may be disposed between the mixing chamber 105 and the storage chamber 103, on the end of the inlet 107. Each of the seals 102 and 104 prevents the contents of its storage chamber (101 or 103) from entering the mixing chamber 105 until each seal is fractured or broken or otherwise compromised. In some embodiments, there may be a single seal instead of two seals as shown. For example, in such embodiments, the inlets 107 and 109 may merge inside the mixing chamber 105 to form a single inlet into the mixing chamber 105, and the single seal may cover the single inlet. In some embodiments, a single seal may be formed in one of the storage chambers (101 or 103) by heat, such as via a heat plate, ultrasonic welding, etc.

In various embodiments, a seal may be made of a polymer film or a metal foil. In an embodiment, a seal may be removed or punctured by the user. In an embodiment, a seal may be a frangible seal. In such an embodiment, the frangible seal may include or may be made of a material that fractures or otherwise compromises upon exposure to sufficient force or pressure, such as the force or pressure exerted on the seal by the contents of one or both of the storage chambers 101 and 103 when external force or pressure is exerted on one or both of the storage chambers 101 and 103. Upon fracturing, puncturing, removing, or otherwise compromising the seal, a pathway is exposed that fluidically couples the mixing chamber 105 and the storage chambers 101 and 103. When the seal is no longer a barrier, the two contents in the two storage chambers 101 and 103 may flow via a respective one of the inlets 109 and 107 and enter the mixing chamber 105. Each of the at least two storage chambers 101 and 103 may be connected to a respective one of the inlets 109 and 107, extending from the mixing chamber 105. As shown in FIG. 2, contents of the first storage chamber 101 and the contents of the second storage chamber 103 may be dispensed from dispenser 100 through an opening 106 as a tooth-whitening composition 110 and onto a separate substrate, such as an applicator, for example a mouth-tray 112.

In another embodiment, the at least two storage chambers may be a first storage chamber and a second storage chamber. In an embodiment the first storage chamber and the second storage chamber are merely separate containers, each with a corresponding lid, and not part of a product dispenser, such as a multi-component product dispenser. Accordingly, the contents of the first storage chamber and the contents of the second storage chamber may be individually removed and mixed on a separate substrate, such as an applicator, for example a mouth-tray.

***Whitening Agent***

In various embodiments, the tooth whitening compositions disclosed herein comprise at least one whitening agent as a main active ingredient. In certain embodiments, the at least one whitening agent is a peroxide compound. As further discussed below, a "whitening agent" is a material which effects whitening of a tooth surface to which it is applied.

As referred to herein, a "peroxide compound" is an oxidizing compound comprising a bivalent oxygen-oxygen group. Peroxide compounds include peroxides and hydroperoxides, such as hydrogen peroxide, peroxides of alkali and alkaline earth metals, organic peroxy compounds, peroxy acids, pharmaceutically-acceptable salts thereof, and mixtures thereof. Peroxides of alkali and alkaline earth metals include lithium peroxide, potassium peroxide, sodium peroxide, magnesium peroxide, calcium peroxide, barium peroxide, and mixtures thereof. Organic peroxy compounds include carbamide peroxide (also known as urea hydrogen peroxide), glyceryl hydrogen peroxide, alkyl hydrogen peroxides, dialkyl peroxides, alkyl peroxy acids, peroxy esters, diacyl peroxides, benzoyl peroxide, and monoperoxy phthalate, and mixtures thereof. Peroxy acids and their salts include organic peroxy acids such as alkyl peroxy acids, and monoperoxyphthalate and mixtures thereof, as well as inorganic peroxy acid salts such as persulfate, dipersulfate, percarbonate, perphosphate, perborate and persilicate salts of alkali and alkaline earth metals such as lithium, potassium, sodium, magnesium, calcium and barium, and mixtures thereof. In various embodiments, the peroxide compound comprises hydrogen peroxide, urea peroxide, sodium percarbonate and mixtures thereof.

Peroxide releasing compounds that may be mentioned for use in the tooth whitening compositions disclosed herein include peroxide containing compounds such as urea peroxide, sodium percarbonate, sodium perborate and polyvinylpyrrolidone-$H_2O_2$ complexes (hereinafter "PVP-$H_2O_2$"). Polyvinylpyrrolidone is also known as poly-N-vinyl-poly-2-pyrrolidone and commonly abbreviated to "PVP". PVP generally refers to a polymer containing vinylpyrrolidone (also referred to as N-vinylpyrrolidone, N-vinyl-2-pyrrolidione and N-vinyl-2-pyrrolidinone) as a monomeric unit. The monomeric unit consists of a polar amide group, four non-polar methylene groups and a non-polar methane group.

Both linear and cross-linked complexes of PVP-$H_2O_2$ are known in the art, and PVP-$H_2O_2$ is considered to be stable in an anhydrous environment. Upon exposure to highly aqueous environments, such as in the oral cavity, the PVP-$H_2O_2$ dissociates into individual species (PVP polymer and $H_2O_2$). In one embodiment, the PVP-$H_2O_2$ complex is 80% by weight polyvinylpyrrolidone and 20% by weight $H_2O_2$.

In alternate embodiments disclosed herein, the at least one whitening agent comprises a liquid peroxide solution. The hydrophobic polymer carrier of the whitening composition provides sufficient stability to permit the use of a liquid hydrogen peroxide. The liquid hydrogen peroxide comprises $H_2O_2$ generally contained in an aqueous water-based solution. In some embodiments, the liquid hydrogen peroxide has a concentration of peroxide to the total solution ranging from about 0.035% to about 17.5%, such as from about 3% to about 10% by weight, which for example may be achieved by adding a 35 wt % aqueous $H_2O_2$ solution at a concentration of from about 0.1 wt % to about 50 wt %, such as about 8 wt % to about 29 wt %, or from about 15 wt % to about 25 wt %. In an embodiment, the tooth whitening composition of the embodiments includes 1% of the whitening agent, which may be in the form of the PVP-$H_2O_2$ complex.

Additionally, at least one stabilizer may be present with a composition that includes the whitening agent. For example, a 3% hydrogen peroxide solution with about 0.1% to about 0.5% of at least one stabilizer may be used. Acetanilide or a similar organic material can also be used with a pyrophosphate stabilizer such as sodium acid pyrophosphate, present in an amount ranging from about 0.1% to about 1.0%, such as about 0.5%.

In certain embodiments, the tooth whitening composition may further comprise at least one agent to enhance release of the peroxide in the oral cavity as a part of the peroxide component whitening agent. In an embodiment, the at least one agent to enhance release of the peroxide of in the oral cavity may be provided with the tooth whitening agent in a first container, with the bleach activator in the second container or in a third container, the contents of which are mixed with the whitening agent and/or bleach activator upon use of the tooth whitening composition. Polypore, which is an allyl methacrylate crosspolymer, available from Amcol health & Beauty Solutions, Inc., is an exemplary enhancing agent.

In various embodiments, the at least one whitening agent is present in the tooth whitening composition in an amount of up to about 35%, including from about 0.035% to 17.5%, such as an amount less than or equal to about 10% and greater than about 0%, including from about 0% to about 0.1%, from about 0.1% to about 10%, or from about 0.1% to about 6%, including an amount of about 1% by weight relative to the total weight of the tooth whitening composition. Therefore, the concentration of tooth whitening agent in a first component disposed in a first storage chamber may be preselected and/or a volume of the first component may be controlled upon discharging from the first storage chamber such that the above amounts of whitening agent are provided in a tooth whitening composition formed by combining the tooth whitening agent and the bleach activator.

In embodiments disclosed herein, the at least one whitening agent may be hydrogen peroxide, and in certain exemplary embodiments, the hydrogen peroxide may be present in the tooth whitening composition in an amount ranging from about 0.1% to about 10% by weight, for example about 0.1% to about 3% by weight, including about 1% and about 2% by weight, relative to the total weight of the composition. Therefore, the concentration of hydrogen peroxide in a first

component disposed in a first storage chamber may be preselected and/or a volume of the first component may be controlled upon discharging from the first storage chamber such that the above amounts of hydrogen peroxide are provided in a tooth whitening composition formed by combining the hydrogen peroxide and the bleach activator.

***Bleach Activator***

Peracids are better oxidants than hydrogen peroxide, and thus provide a superior whitening benefit. The whitening agent, such as a peroxide as described above, which may be the primary active ingredient of a tooth whitening composition of an embodiment, may thus be reacted with a bleach activator, such as an activated ester, to form the tooth whitening composition, which may be a peracid and which may be used for enhancing bleaching effect.

In an embodiment, the tooth whitening composition-which may be anhydrous-may be exposed to an aqueous environment such as during application of the tooth whitening composition in the mouth.

In an embodiment, the tooth whitening composition thus be formed by combining a first component that may include a whitening agent and a second component that may include a bleach activator. In order to keep each of the whitening agent and bleach activator stable, or to keep them from reacting with one another, they are each be kept in separate storage containers until time of use at which point they are combined to form a tooth whitening composition. However, for maximum reaction between the whitening agent and the bleach activator to form the tooth whitening composition, the first component, the second component or both the first component and second component may individually include at least one buffering agent in addition to the whitening agent and the bleach activator, respectively. The whitening agent may also be provided as an aqueous composition. Accordingly, when the first component comprising the aqueous composition of the whitening agent (and possibly at least one of a first buffering agent) is combined with the second component comprising the bleach activator (and possibly at least one of a second buffering agent that may the same or different than the first buffering agent), the combination may be provided at an appropriate pH for maximizing the reaction product, such as the tooth whitening composition. Exposure to an additional aqueous environment, such as the oral cavity, may further provide for an appropriate pH for maximizing the reaction product after combining the two components and thereby increase whitening effect.

The bleach activator may react with the whitening agent at an appropriate pH, such as within the range of 3 pH to 12 pH. Exemplary pH values may include a pH of greater than about 5.5 pH and less than or equal to about 9 pH, for example in a range of about 5.5 pH to about 6 pH, including a pH of greater than about 6 pH and less than or equal to about 9 pH. In an embodiment, the bleach activator may react with the peroxide at a pH in the range of about 6 pH to about 8 pH, for example a pH of about 7.5 pH. While stored in, for example, a first storage chamber, the first component comprising the whitening agent may be kept at an acidic pH (i.e., less than about 7pH). Meanwhile the second composition comprising the bleach activator may be anhydrous and, therefore, may not be characterized based on a corresponding pH. However, the second component may also include a buffering agent that, upon mixing the first component and the second component to form a tooth whitening composition, provides for higher pH for the tooth whitening composition as compared to a pH of the first component stored in the first storage chamber.

The bleach activator may react with the peroxide according to general reaction Scheme 1:

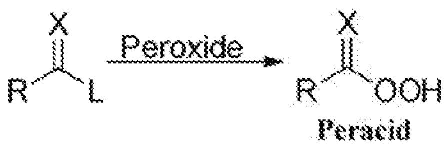


Scheme 1

where the bleach activator may be any compound, for example an activated ester, as represented by formula I:

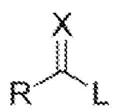

(I)

wherein L may be any leaving group, including -O-R' or -NR"R', where R' may be any alkyl or aryl group wherein the alkyl group can include repeat units of C1 to C30, for example, C1 to C8, and R" may be hydrogen or any alkyl or aryl group wherein the alkyl group can include repeat units of C1 to C30, for example, C1 to C8. Additionally in formula (I), R may be a branched or straight chained alkyl group, including repeat units of C1 to C30, for example, C1 to C8, and X may include O, N or S. For example, the bleach activator of the embodiments may be represented by formula II:

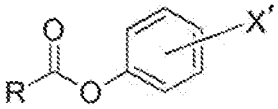

(II)

where R is as described above for formula (I) and X' may be an electron withdrawing group (ewg) comprising a counter-ion such as a metal or organic counter-ion. In examples, X' may be selected from -COOH, -COONa, -$SO_3H$, and - $SO_3Na$.

For reference, exemplary bleach activators are N- or O Acyl compounds, tertraacetylglycouril, N-acetyl N-[4-(triethylammoniomethyl) benzoyl] caprolactam chloride, sodium acetoxy-benzene sulfonate,sodium-benzoyloxy benzene sulfonate, sodium-lauroyloxy-benzene sulfonate, sodiumisononanoyloxy benzene sulfonate, acylated sugar derivatives, pentaglucose, tetra acetyl ethylenediamine and sodium nonanoyloxybenzenesulfonate (SNOBS or simply, NOBS). One commercially available bleach activator of the current invention is FUTURECHEM® Nonanoic acid, sulfophenyl ester, sodium salt (available from FutureFuel Chemical Company of Batesville, AR).

The bleach activator of the invention comprises sodium nonanoyloxybenzenesulfonate and so the reaction with peroxide forms peroxynonanoic acid (also known as pernonanoic acid) (PNA) as represented by reaction Scheme 2:

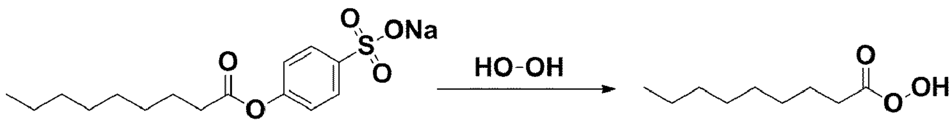


**SNOBS** **Pernonanoic Acid** Scheme 2

The bleach activator of the embodiments may be present in the tooth whitening composition in an amount ranging from about 0.1% to about 25% by weight, such as about 1.5% to about 5% by weight, for example about 2.5% by weight, relative to the total weight of the composition. Therefore, the concentration of bleach activator in a second component disposed in a second storage chamber may be preselected and/or a volume of the second component may be controlled upon discharging from the second storage chamber such that the above amounts of bleach activator are provided in a tooth whitening composition formed by combining the whitening agent and the bleach activator.

### *Buffering Agent*

As discussed above, for maximum reaction between the whitening agent and the bleach activator to form the tooth whitening composition, the first component, the second component or both the first component and second component may individually include at least one buffering agent in addition to the whitening agent and the bleach activator. In an embodiment, the second component comprises the buffering agent. The buffering agent may be provided at whatever composition provides for a higher pH for the composition. The buffering agent may be the same or different than an anti- calculus agent, such as the materials described below for the anti-calculus agent of the embodiments. For example, the buffering agent may be one or more selected from TSPP, TKPP, STPP, sodium phosphate dibasic and sodium hydroxide.

### *Additional Ingredients*

As previously described, many other ingredients may further be included in the whitening compositions of the present invention, and include surfactants, thickening agents, flavoring agents, sweetening agents, desensitizing agents, anti-microbial agents, anti-caries agents, anti-calculus agents, anti-inflammatory agents, vitamins, pigments and coloring agents, enzymes, preservatives, abrasive agents, and tartar control agents, for example. Any one or more of such additional ingredients may be included in the first component that includes the tooth whitening agent and/or in the second component that includes the bleach activator. The one or more additional ingredients that may be included in the first component may be the same or different ones of the one or more additional ingredients in the second component.

Any orally acceptable surfactant, most of which are anionic, nonionic or amphoteric, can be used. Suitable anionic surfactants include without limitation water-soluble salts of $C_{8-20}$ alkyl sulfates, sulfonated monoglycerides of $C_{8-20}$ fatty acids, sarcosinates, taurates and the like. Illustrative examples of these and other classes include sodium lauryl sulfate, sodium cocoyl monoglyceride sulfonate, sodium lauryl sarcosinate, sodium lauryl isoethionate, sodium laureth carboxylate and sodium dodecyl benzenesulfonate. Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation derivatives of $C_{8-20}$ aliphatic secondary and tertiary amines having an anionic group such as carboxylate, sulfate, sulfonate, phosphate or phosphonate. A suitable example is cocoamidopropyl betaine.

In some embodiments, one or more surfactants may be present in a total amount of from about 1.8% to about 2% w/w. In some embodiments, one or more surfactants may be present in a total amount of from about 1.9% to about 2% w/w. In some embodiments, one or more surfactants may be present in a total amount of about 2% w/w.

In some embodiments, the composition optionally comprises a thickening agent. Any orally acceptable thickening agent can be used, including without limitation carbomers, also known as carboxyvinyl polymers, carrageenans, also known as Irish moss and more particularly -carrageenan (iota-carrageenan), high molecular weight polyethylene glycols (such as CARBOWAX, available from The Dow Chemical Company), cellulosic polymers such as hydroxyethylcellulose, carboxymethylcellulose (CMC) and salts thereof, *e.g.,* CMC sodium, natural gums such as karaya, xanthan, gum arabic and tragacanth, colloidal magnesium aluminum silicate, and colloidal and/or fumed silica and mixtures of the same. In some embodiments, the one or more optional thickening agents are present in a total amount of about 0.1% to about 90% w/w. In some embodiments, the one or more optional thickening agents are present in a total amount of about 1% to about 50% w/w. In some embodiments, the one or more optional thickening agents are present in a total amount of about 5% to about 35% w/w.

In certain embodiments disclosed herein, the tooth whitening composition may further comprise at least one flavoring agent. As an example, at least one flavoring agent may be included in the first component that includes the tooth whitening agent and/or in the second component that includes the bleach activator. The same or different ones of an at least one flavoring agent may be included in the first component than in the second component.

The at least one flavoring agent, may, for example, be selected from essential oils, as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Of these, the most commonly employed are the oils of peppermint, spearmint and wintergreen. The flavoring agent may be incorporated in the tooth whitening compositions disclosed herein at a concentration ranging from 0.01% to about 2% by weight, such as about 0.1% to about 0.6% by weight.

In embodiments where the tooth whitening composition is sweetened, at least one sweetening agent may be used as an alternative or as a complement to the at least one flavoring agent. Suitable sweetening agents may be water-soluble and include, for example, sodium saccharin, sodium cyclamate, xylitol, perillartien, D-tryptophan, aspartame, dihydrochalcones and the like. The at least one sweetening agent may be present in the tooth whitening composition in an amount ranging from about 0.01% to about 1% by weight, such as about 0.3%.

Exemplary antimicrobial agents may include those typically used in oral care compositions, such as Thymol, benzyl alcohol, Triclosan, chlorhexidine, stannous salts including copper-, zinc-and stannous salts such as zinc oxide, zinc lactate, zinc citrate, zinc sulfate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2'methylenebis-(4-chloro-6-bromophenol).

Some embodiments of the present disclosure comprise a dental abrasive or combination of dental abrasive agents. As used herein, the term "abrasive" or "abrasive agent" also includes materials commonly referred to as "polishing agents." Any orally acceptable abrasive can be used, but typically, type, fineness (particle size) and amount of abrasive should be selected so that tooth enamel is not excessively abraded in normal use of the composition. Suitable abrasives include without limitation silica (in the form of silica gel, hydrated silica or precipitated silica), alumina, insoluble phosphates, calcium carbonate, resinous abrasives such as urea-formaldehyde condensation products and the like.

Among insoluble phosphates useful as abrasives are orthophosphates, polymetaphosphates and pyrophosphates. Illustrative examples are dicalcium orthophosphate dihydrate, calcium pyrophosphate, n-calcium pyrophosphate, tricalcium phosphate, calcium polymetaphosphate and insoluble sodium polymetaphosphate.

Average particle size of an abrasive, if present, is generally about 0.1 to about 30 $\mu$m for example about 1 to about 20 $\mu$m or about 5 to about 15 $\mu$m. In some embodiments, one or more abrasives are present in an amount of about 0.1 % to about 40% w/w. In some embodiments, the abrasive is calcium pyrophosphate. In some embodiments, the calcium pyrophosphate is present in an amount from about 5% to about 50% w/w.

In various embodiments of the present disclosure, the stable whitening dentifrice composition comprises an anticalculus agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. In some embodiments, the anticalculus agent is present in an amount of about 0.1% to about 30% w/w. In some embodiments, the stable whitening dentifrice composition comprises a mixture of anticalculus agents. In some embodiments, tetrapotassium pyrophosphate (TKPP), tetrasodium pyrophosphate (TSPP) and sodium tripolyphosphate (STPP) are used as the anticalculus agents. In some embodiments, the anticalculus agent comprises about 1% to about 2% w/w TSPP, and about 0% to about 7% w/w STPP.

Another desirable component of the present compositions is a synthetic anionic polymeric polycarboxylate (SAPP), which acts as a stabilizer for the polyphosphate anti-tartar agent and may help to block access of painful or

pain-causing materials, such as sugars, to the tooth nerves.

Exemplary anti-inflammatory agents may include those typically used in oral care compositions, such as ibuprofen, flurbiprofen, aspirin, and indomethacine. Exemplary anti-caries agents may include ingredients such as sodium-, calcium-, magnesium-and stannous fluoride, aminefluorides, disodium monofluorophosphate and sodium trimetaphosphate. Exemplary vitamins may include ingredients such as Vitamin C. Exemplary desensitizing agents may include ingredients such as potassium citrate, potassium chloride, potassium tartrate, potassium bicarbonate, potassium oxalate, potassium nitrate and strontium salts. Exemplary anti-calculus agents may include ingredients such as pyrophosphate salts including the mono, di, tri and tetra alkali metal and ammonium pyrophosphate and tripolyphosphate salts. Exemplary enzymes may include glucoamylase.

Some embodiments provide compositions wherein at least one of ingredients is a fluoride ion source selected from stannous fluoride, sodium fluoride, potassium fluoride, sodium monofluorophosphate, sodium fluorosilicate, ammonium fluorosilicate, amine fluoride, and ammonium fluoride.

Also disclosed herein are methods for whitening a surface of a tooth in an oral cavity of a human or other animal subject. One such method comprises (a) applying a tooth whitening composition as disclosed herein to the tooth surface to be whitened for a plurality of minutes per day; and (b) repeating step (a) for multiple days to thereby whiten the teeth. Also disclosed herein is a method for whitening a surface of a tooth comprising: applying, to the surface of the tooth, a composition comprising at least one whitening agent and at least one bleach activator comprising an activated ester. Another such method comprises (a) combining a tooth whitening agent disposed in a first storage chamber with a bleach activator disposed in a second storage chamber to form a tooth whitening composition, (b) applying the tooth whitening composition as disclosed herein to the tooth surface to be whitened for a plurality of minutes per day; and (c) repeating steps (a)-(b) for multiple days to thereby whiten the teeth. Also disclosed herein is a method for whitening a surface of a tooth comprising: combining a tooth whitening agent disposed in a first storage chamber with a bleach activator disposed in a second storage chamber to form a tooth whitening composition; applying, to the surface of the tooth, the tooth whitening composition comprising at least one whitening agent and at least one bleach activator

Exemplary methods disclosed herein comprise contacting the tooth whitening composition with the surface of the tooth. The contacting may occur for a duration of time sufficient to satisfactorily affect whitening of the teeth. Thus, the contacting occurs for a sufficient period of time to at least partially whiten teeth. This can be a period of time ranging from about 1 minute to about 2 hours or longer. In certain embodiments, the contacting is for a period of time ranging from about 1 minute to about 5 minutes, from about 1 minute to about 45 minutes, from about 5 minutes to about 45 minutes, or from about 5 minutes to about 30 minutes.

In certain embodiments disclosed herein, a tooth whitening composition, which may be substantially non-aqueous or may be aqueous, may be effective over a longer period of time, since it is not significantly diluted or washed away in the oral cavity during the treatment time. The tooth whitening composition can be removed as and when required, at will, by an employment of standard oral hygiene procedures such as brushing or by rinsing with an alcoholic mouthwash. While in place, the composition may release agents contained therein at a slow, relatively constant rate and in concentration sufficient to effect stain removal from or whitening of the teeth.

Further disclosed herein are methods of making a tooth whitening composition. In an example, a tooth whitening composition of an embodiment may be prepared by adding and mixing the ingredients of the composition in a suitable vessel, such as a stainless steel tank provided with a mixer. In the preparation of the tooth whitening composition, the ingredients may be added to the mixer in the following order: hydrophobic polymer component, such as the silicone based pressure sensitive polymer; peroxide whitening agent; adhesion enhancing agents, including fumed silica and petrolatum; and any desired flavoring or sweetener. The ingredients may then be mixed to form a homogenous dispersion/solution. In another example, a tooth whitening composition of an embodiment may be prepared by adding and mixing the ingredients of each of a first component including a whitening agent and/or the ingredients of a second component including a bleaching agent separately in a suitable vessel, such as a stainless steel tank provided with a mixer. In the preparation of the first component including a whitening agent and/or the second component including a bleach activator, the ingredients of each of the first component and the second component may be separately added to a mixer: i) for the first component: hydrophobic polymer component, such as the silicone based pressure sensitive polymer; peroxide whitening agent; adhesion enhancing agents, including fumed silica and petrolatum; and any desired flavoring or sweetener; and ii) for the second component: hydrophobic polymer component, such as the silicone based pressure sensitive polymer, bleach activator, adhesion enhancing agents, including fumed silica and petrolatum; and any desired flavoring or sweetener. The ingredients of each of the first component and in the second component may then be separately mixed to form a homogenous dispersion/solution for the first component and/or the second component.

A tooth whitening composition of an embodiment comprising at least one whitening agent and at least one bleach activator, for example, a first component comprising a whitening agent, the second component comprising a bleach activator wherein tooth whitening composition formed by combining the first component and the second component, may be prepared in the form of a flowable viscous liquid dispersion, such as a gel. In an example, is the tooth whitening composition may be applied directly onto the user's teeth such as by painting the teeth with a soft applicator

brush or applying by a mouth-tray, including placing the first component and the second component on the mouth tray and then inserting into the user's mouth. Application by the user leaves a coating of the composition on the teeth. Contact with an aqueous environment at the proper pH, such as water in an aqueous composition for the first component comprising the whitening agent, or such as saliva in the user's mouth, causes the at least one whitening agent, for example, $H_2O_2$, to dissociate and react with the at least one bleach activator, providing prolonged whitening treatment of the tooth sites.

**EXAMPLES**

**Example 1** - **Conventional Tooth Whitening Composition (12% Hydrogen Peroxide, 0% Sodium nonanoyloxybenzenesulfonate)**

A conventional tooth-whitening composition having 12% hydrogen peroxide whitening agent was prepared without any bleach activator of the embodiments by mixing the components of Table 1, below. The resulting formulation, including a listing of ingredients listed by wt% is provided in Table 1.

TABLE 1

| INGREDIENT | Weight % |
|---|---|
| Thickener | 3 |
| Potassium Nitrate | 3 |
| Water and minors (color, fragrance, sweetener, flavor) | Q.S. |
| Humectant | 30 |
| 35% Hydrogen Peroxide | 34.29 |
| Sodium Hydroxide | 1.85 |

**Example 2** - **Preparation of Compositions with 5% bleach activator**

To assess the ability of SNOBS to enhance the whitening efficacy of hydrogen peroxide, a first SNOBS-based composition comprising bleach activator sodium nonanoyloxybenzenesulfonate was prepared according to the composition of Table 2.

TABLE 2

| INGREDIENT | Weight % |
|---|---|
| Non-ionic surfactant | 45.5 |
| Humectant | 22.5 |
| Thickening agents | 12 |
| Sodium tripolyphosphate, tetra sodium polyphosphate | 15 |
| SNOBS | 5 |

**Example 3 - Forming a Mixed Gel Composition Comprising Whitening Agent and Bleach Activator (6% Whitening Agent; 2.5% Bleach Activator)**

A mixed gel composition was prepared by combining equal amounts of an aqueous 12% hydrogen peroxide gel with the 5% SNOBS gel to form a composition having a concentration after mixing of 6% hydrogen peroxide and 2.5% SNOBS. Example 4 - Measuring peracid concentration versus time.

**Example 4** - **Peracid Generation**

To quantify the amount of pernonanoic acid (PNA) generated by mixing the peroxide gel and SNOBS gel, the Karst assay (Karst et al., Anal. Chem. 1997, 69, 3623-3627) was utilized. Briefly, equal parts of 12% hydrogen peroxide gel and 5% SNOBS gel were mixed. 20 μl of the mixture was added to a phosphoric acid solution at timed intervals to

stop the generation of peracid, the results of which are summarized in Table 3. This solution was then allowed to react with an excess of methyl-p-tolylsulfide (MTS) to generate methyl-p-tolylsulfoxide (MTSO). MTSO is then quantified by HPLC and the concentration of PNA is back calculated. Table 4 shows the concentration of PNA generated.

TABLE 3

| Time / min | 0 | 2 | 5 | 15 | 30 |
|---|---|---|---|---|---|
| % PNA | 0 | 0.32 | 0.53 | 0.43 | 0.59 |

Results show that a significant amount (0.6% at 30 minutes) of pernonanoic acid generated by mixing SNOBS gel with hydrogen peroxide gel. Peracid are better oxidants than hydrogen peroxide and thus results in improved whitening.

**Example 5** - **In vitro bovine enamel testing**

Bovine teeth (mounted on acrylic resin) were brushed with an abrasive toothpaste to achieve baseline L values between 60 and 65. Eight bovine teeth were separated into two groups and baseline L* a* b* values were recorded using a hand held spectrophotometer. Each tooth was placed in a 24 well plate and soaked in artificial saliva for 30 minutes. 100 μg of the mixed gel composition of Example 2 was spread around the surface of the bovine teeth and left for 30 minutes. The gel was then wiped from the bovine teeth surface.

L a b values for the bovine teeth were gathered. These values were used to calculate the change in whiteness for each bovine tooth after treatment as compared to baseline ($W^*_{baseline}$) as shown in FIG. 3. The bovine teeth were then soaked in artificial saliva for 20 minutes. The process was repeated 7 times to mimic once-per-day use for one week. The calculated change in whiteness is reported here based on a W* value, which is a measure of the overall color change relative to pure white. It should be noted that the more negative value of ΔW*, the closer the tooth color is to white, where W* is determined as:

$$W^* = ((a^*-0)^2 + (b^*-0)^2 + (L^* - 100)^2)^{1/2}$$

and

$$\Delta W^* = W^*_{treated} - W^*_{baseline}.$$

Whitening efficacy was tested using the sample having 2.5 wt% sodium nonanoyloxybenzenesulfonate (SNOBS) as compared to a sample having 0 wt% SNOBS as shown in FIG. 3. After 7 treatments on bovine teeth, significant differences in whitening efficacy were obtained. The modified tooth-whitening composition containing 2.5 % SNOBS delivered superior whitening at faster rate compared to the composition containing hydrogen peroxide alone. As shown in FIG. 3, seven treatments with 6% HP has the equivalent result as approximately 3 treatments in the presence of the 2.5% SNOBS formulation.

Table 4 summarizes increased whitening (ΔW*) effect of bovine tooth samples treated with the 2.5 wt % of SNOBS-containing composition paste versus the control whitening paste with 6% hydrogen peroxide (0% SNOBS) over the course of 7 treatments.

TABLE 4

| Treatment | **1** | **2** | **3** | **4** | **5** | **6** | **7** |
|---|---|---|---|---|---|---|---|
| **6%HP** | -5.55 | -10.73 | -14.14 | -16.10 | -16.722 | -17.65 | -18.18 |
| **6% HP & 2.5% SNOBS** | -10.24 | -16.14 | -17.90 | -19.48 | -19.76 | -19.95 | -21.28 |

**Example 6 - SNOBS Stability**

SNOBS was included an anhydrous formulation according to a composition based on the ingredients shown in Table 5.

TABLE 5

| INGREDIENT | % |
|---|---|
| Non-ionic Surfactant | 33 |
| Thickening agents | 40 |
| Humectant | 10 |
| Abrasives (fumed silica) | 5 |
| TSPP | 2 |
| SNOBS | 10 |

The composition of Table 5 was aged at 40°C and at room temperature for 8 weeks and 13 weeks respectively. The percentage of SNOBS remaining was quantified by HPLC. The results in Table 6 indicate that there is sufficient SNOBS remaining in the composition after aging at both room temperature and at 40°C to be efficacious as a booster for the peroxide component in a tooth whitening composition.

TABLE 6

| Time / Aging Temp. | % SNOBS | % Loss of SNOBS |
|---|---|---|
| Initial | 9.6 | - |
| 4 weeks / 40 °C | 936 | 3.1 |
| 8 weeks / 40 °C | 8.99 | 6.4 |
| 13 weeks / Room Temperature (RT) | 9.21 | 4.06 |

**Example 7 - Conventional Tooth Whitening Composition (0% Sodium nonanoyloxybenzenesulfonate)**

A conventional tooth-whitening toothpaste having 1% hydrogen peroxide whitening agent was prepared without any bleach activator by mixing the components of Table 1, below. The resulting formulation, including a listing of ingredients listed by wt% is provided in Table 7.

TABLE 7

| INGREDIENT | Weight % |
|---|---|
| Humectant | 44.16 |
| Abrasive System II | 18.38 |
| Thickening agents | 23.38 |
| Cross-linked PVP complexed with Hydrogen Peroxide | 5.50 |
| minors (color, fragrance, flavor) | Q.S. |
| Tetrasodium Pyrhophosphate - Fine FCC | 2.00 |
| Sodium Lauryl Sulfate Powder | 2.00 |
| Sodium Acid Pyrophosphate | 0.90 |
| Sodium Monofluorophosphate - USP | 0.76 |
| Butyl ated hydroxytoluene, FCC grade | 0.03 |
| Total | 100.00 |

**Example 8 - Tooth Whitening Toothpaste with Bleach Activator (1% Whitening Agent; 2.5% Bleach Activator)**

Bleach activator sodium nonanoyloxybenzenesulfonate (SNOBS) was added to the commercial (1% hydrogen peroxide) tooth whitening formulation at 2.5% of Table 1 by decreasing the amount of propylene glycol based so as to

arrive at the formulation listed in Table 8.

TABLE 8

| INGREDIENT | Weight % |
|---|---|
| Humectant | 41.66 |
| Abrasives (calcium pyrophosphate - FCC, fumed silica) | 18.38 |
| Thickening agents | 23.38 |
| Cross-linked PVP complexed with Hydrogen Peroxide | 5.50 |
| minors (color, fragrance, flavor) | Q.S. |
| Tetrasodium Pyrhophosphate - Fine FCC | 2.00 |
| Sodium Lauryl Sulfate Powder | 2.00 |
| Sodium Acid Pyrophosphate | 0.90 |
| Sodium Monofluorophosphate - USP | 0.76 |
| Butylated hydroxytoluene, FCC grade | 0.03 |
| Nonanoic acid, sulfophenyl ester, sodium salt (1:1) | 2.50 |
| Total | 100.00 |

**Example 9** - **In vitro bovine enamel testing**

Bovine teeth (mounted on acrylic resin) were brushed with an abrasive toothpaste to achieve baseline L values between 60 and 65. Bovine teeth were separated into 3 groups and baseline L* a* b* values were recorded using a hand held spectrophotometer. Each grouping of bovine teeth was placed in a designated glass beaker for subsequent treatment with toothpaste containing 0% (Conventional Tooth whitening formulation), 2.5% (Tooth whitening formulation of Table 8), and 5% SNOBS as shown in Table 9 (further addition of bleach activator to formulation of Table 8 while decreasing the amount of propylene glycol), respectively.

TABLE 9

| INGREDIENT | Weight % |
|---|---|
| Humectant | 39.16 |
| Abrasives | 18.38 |
| Thickening agents | 23.38 |
| Cross-linked PVP complexed with Hydrogen Peroxide | 5.500 |
| minors (color, fragrance, flavor) | Q.S. |
| Tetrasodium Pyrophosphate - Fine FCC | 2.00 |
| Sodium Lauryl Sulfate Powder | 2.00 |
| Sodium Acid Pyrophosphate | 0.90 |
| Sodium Monofluorophosphate-USP | 0.76 |
| Butyl ated hydroxytoluene, FCC grade | 0.03 |
| Nonanoic acid, sulfophenyl ester, sodium salt (1:1) | 5.00 |
| Total | 100.00 |

Samples containing 7g of a whitening toothpaste with 0 wt%, 2.5 wt% and 5 wt% of SNOBS bleach activator were separately prepared by mixing with 7ml of artificial saliva. The samples were quickly mixed and poured into the designated beakers containing the bovine teeth described above. The beakers were placed in a shaker and left to shake for 2 minutes. The bovine teeth from each beaker were then rinsed with water until the toothpaste was washed away.

The shaking and rinsing steps were repeated 14 times to mimic one week of toothpaste usage (i.e., 14 treatments) for each of the beakers. L a b values for the bovine teeth were gathered after the 7th and 14th treatment ($W^*_{treatment}$). These values were used to calculate the change in whiteness for each bovine tooth after treatment as compared to baseline ($W^*_{baseline}$) The calculated change in whiteness is reported here based on a W* value, which is a measure of the overall color change relative to pure white. It should be noted that the more negative value of ΔW*, the closer the tooth color is to white, where W* is determined as:

$$W^* = ((a^*-0)^2 + (b^*-0)^2 + (L^* - 100)^2)^{1/2}$$

and

$$\Delta W^* = W^*_{treated} - W^*_{baseline}.$$

Whitening efficacy was tested using the sample having 2.5 wt% sodium nonanoyloxybenzenesulfonate (SNOBS) added to a conventional, commercial tooth-whitening toothpaste (1% hydrogen peroxide). After 14 two minute treatments on bovine teeth, significant differences in whitening efficacy were obtained. The modified tooth-whitening toothpaste containing 2.5 % SNOBS showed a 64% increase in whitening versus commercial tooth-whitening toothpaste that does not include any of the SNOBS as shown in FIG. 4. Overall, the data in FIG. 4 shows SNOBS provided a significant boost in whitening.

Table 10 summarizes increased whitening (ΔW*) effect of bovine tooth samples treated with 5% and 2.5 wt % of SNOBS-containing whitening paste versus a control whitening paste with (0% SNOBS).

TABLE 10

| | **7 Treatments** | **14 Treatments** |
|---|---|---|
| **0% SNOBS (1% HP)** | -1.14 | -2.38 |
| **2.5% SNOBS (1% HP)** | -2.66 | -3.91 |
| **5% SNOBS (1% HP)** | -2.31 | -4.37 |

**Example 10 - Stability of 1% HP tooth-whitening system with 2.5% SNOBS toothpaste in 60C Ageing**

Batches of a comparative 1% HP tooth-whitening system (without a bleach activator) and a tooth-whitening system comprising 1% HP and 2.5 % sodium nonanoyloxybenzenesulfonate (SNOBS) bleach activator were placed in an oven at 60°C oven. Hydrogen peroxide content was measured initially, after 1 week, and after 2 weeks, the results of which are shown in FIG. 5.

The amount of hydrogen peroxide remaining in each sample was determined by reacting hydrogen peroxide in the toothpaste with potassium iodide in an acid media to liberate iodine. The liberated iodine was then titrated with standard sodium thiosulfate via manual titration. The percent hydrogen peroxide was calculated with equation 1 as follows:

$$\% \text{ Peroxide} = [(A \times N \times C) / (D)] \times 100 / \text{Sample Volume (mL)},$$

where A is the volume of sodium thiosulfate (in mL), N is Normality of Sodium Thiosulfate solution, C is meq wt of oxidizing agent (e.g.., milliequivalent weight of hydrogen peroxide in standard sodium thiosulfate titration - 0.01701), and D is the sample volume (in mL).

After 1 week, the comparative tooth-whitening system (1% HP) with no bleach activator showed a slight decrease in the amount of peroxide (from about 1.0% initially to about 0.91% at week 1), and almost no subsequent change at week 2 (from about 0.91% at week 1 to about 0.905% at week 2). Similarly, the exemplary formulation with 1% HP and 2.5% SNOBS showed an acceptable decrease in the amount of peroxide (from about 0.950% initially to about 0.800% at week 1), and an acceptable subsequent change at week 2 (from about 0.800% at week 1 to about 0.730% at week 2). This 60°C aging study shows that hydrogen peroxide will maintain its stability even in the presence of 2.5% SNOBS.

## Claims

1. A multi-component product dispenser containing a tooth whitening system, comprising:

   at least one whitening agent in a first storage chamber; and
   at least one bleach activator in a second storage chamber, separate from the first storage chamber, wherein the at least one bleach activator comprises sodium nonanoyloxybenzenesulfonate.

2. The multi-component product dispenser of any claim 1, wherein the at least one whitening agent comprises hydrogen peroxide.

3. The system of any one of the preceding claims, further comprising a mixing chamber, connected to both the first storage chamber and the second storage chamber, that receives and sufficiently mixes contents from the first storage chamber and the second storage chamber.

4. A method for whitening a surface of a tooth comprising:

   combining at least one whitening agent and at least one bleach activator to form a tooth whitening composition, and
   contacting the surface of the tooth with a tooth whitening composition for a duration of time sufficient to effect whitening of the surface of the tooth;
   wherein the at least one bleach activator comprises sodium nonanoyloxybenzenesulfonate.

5. The method of claim 4, wherein in the tooth whitening composition, the at least one bleach activator is present in an amount greater than an amount of the at least one whitening agent.

6. The method of claim 4 or claim 5, wherein in the tooth whitening composition, the at least one whitening agent is present in an amount of greater than about 0 wt% and less than about 10%.

7. The method of any one of claims 4 to 6, wherein the tooth whitening composition comprises an aqueous component.

8. The method of any one of claims 4 to 7, wherein the tooth whitening composition comprises a gel.

9. The method of any one of claims 4 to 8, wherein the first storage chamber and the second storage chamber are storage chambers of an oral care product dispenser.

10. The method of any one of claims 4 to 9, wherein the first storage chamber and the second storage chamber comprise a portion of an oral care product dispenser, wherein the oral care product dispenser further comprises a mixing chamber connected to both the first storage chamber and the second storage chamber, and wherein the combining comprises at least partially mixing the at least one whitening agent and the at least one bleach activator in the mixing chamber.

11. The method of any one claims 4 to 10, wherein the combining comprises mixing the at least one whitening agent and the at least one bleach activator on a surface of a mouth-tray.

12. The method of any one of claims 4 to 11, further comprising at least partially filling the first chamber, the second chamber or both the first and second chambers with at least one of flavoring agents, sweeteners, desensitizing agents, anti-microbial agents, anti-caries agents, anti-calculus agents, anti-inflammatory agents, vitamins, pigments and coloring agents, and preservatives.

## Patentansprüche

1. Mehrkomponenten-Produktspender, der ein Zahnaufhellungssystem enthält, umfassend:

   mindestens ein Aufhellungsmittel in einer ersten Vorratskammer; und
   mindestens einen Bleichaktivator in einer zweiten Vorratskammer, getrennt von der ersten Vorratskammer, wobei der mindestens eine Bleichaktivator Natrium-Nonanoyloxybenzol-Sulfonat umfasst.

2. Mehrkomponenten-Produktspender nach einem beliebigen Anspruch 1, wobei das mindestens eine Aufhellungsmittel Wasserstoffperoxid umfasst.

3. System nach einem beliebigen der vorhergehenden Ansprüche, weiterhin umfassend eine Mischkammer, die sowohl mit der ersten Vorratskammer als auch der zweiten Vorratskammer verbunden ist, die Inhalte aus der ersten Vorratskammer und der zweiten Vorratskammer aufnimmt und hinreichend vermischt.

4. Verfahren zum Aufhellen einer Zahnoberfläche, umfassend:

   Kombinieren von mindestens einem Aufhellungsmittel und mindestens einem Bleichaktivator um eine Zahnaufhellungszusammensetzung zu bilden, und
   Inkontaktbringen der Zahnoberfläche mit einer Zahnaufhellungszusammensetzung für einen Zeitraum, der ausreicht, um eine Aufhellung der Zahnoberfläche zu bewirken;
   wobei der mindestens eine Bleichaktivator Natrium-Nonanoyloxybenzol-Sulfonat umfasst.

5. Verfahren nach Anspruch 4, wobei in der Zahnaufhellungszusammensetzung der mindestens eine Bleichaktivator in einer Menge vorhanden ist, die größer ist als eine Menge von dem mindestens einem Aufhellungsmittel.

6. Verfahren nach Anspruch 4 oder Anspruch 5, wobei in der Zahnaufhellungszusammensetzung das mindestens eine Aufhellungsmittel in einer Menge von mehr als etwa 0 Gew.-% und weniger als etwa 10 % vorhanden ist.

7. Verfahren nach einem beliebigen der Ansprüche 4 bis 6, wobei die Zahnaufhellungszusammensetzung eine wässrige Komponente umfasst.

8. Verfahren nach einem beliebigen der Ansprüche 4 bis 7, wobei die Zahnaufhellungszusammensetzung ein Gel umfasst.

9. Verfahren nach einem beliebigen der Ansprüche 4 bis 8, wobei die erste Vorratskammer und die zweite Vorratskammer Vorratskammern eines Mundpflege-Produktspenders sind.

10. Verfahren nach einem beliebigen der Ansprüche 4 bis 9, wobei die erste Vorratskammer und die zweite Vorratskammer einen Teil eines Mundpflege-Produktspenders umfassen, wobei der Mundpflege-Produktspenders weiterhin eine Mischkammer umfasst, die sowohl mit der ersten Vorratskammer als auch der zweiten Vorratskammer verbunden ist, und wobei das Kombinieren mindestens teilweises Mischen des mindestens einen Aufhellungsmittels und des mindestens einen Bleichaktivators in der Mischkammer umfasst.

11. Verfahren nach einem beliebigen der Ansprüche 4 bis 10, wobei das Kombinieren das Mischen des mindestens einem Aufhellungsmittel und des mindestens einen Bleichaktivators auf einer Oberfläche einer Mundschiene umfasst.

12. Verfahren nach einem beliebigen der Ansprüche 4 bis 11, weiterhin umfassend das mindestens teilweise Füllen der ersten Kammer, der zweiten Kammer oder sowohl der ersten als auch der zweiten Kammer mit mindestens einem von Geschmacksstoffen, Süßungsmitteln, Desensibilisierungsmitteln, antimikrobiellen Mitteln, Anti-Karies-Mitteln, Anti-Zahnstein-Mitteln, entzündungshemmenden Mitteln, Vitaminen, Pigmenten und Farbstoffen sowie Konservierungsmitteln.

**Revendications**

1. Distributeur de produit à plusieurs composants contenant un système de blanchiment des dents, comprenant :

   au moins un agent de blanchiment dans une première chambre de stockage ; et
   au moins un activateur de blanchiment dans une seconde chambre de stockage, séparée de la première chambre de stockage, dans lequel l'au moins un activateur de blanchiment comprend du nonanoyloxybenzènesulfonate de sodium.

2. Distributeur de produit à plusieurs composants selon l'une quelconque de la revendication 1, dans lequel l'au moins un agent de blanchiment comprend du peroxyde d'hydrogène.

**3.** Système selon l'une quelconque des revendications précédentes, comprenant en outre une chambre de mélange, reliée à la fois à la première chambre de stockage et à la seconde chambre de stockage, qui reçoit et mélange suffisamment le contenu de la première chambre de stockage et de la seconde chambre de stockage.

**4.** Procédé pour blanchir une surface d'une dent comprenant :

la combinaison d'au moins un agent de blanchiment et d'au moins un activateur de blanchiment pour former une composition de blanchiment des dents, et
la mise en contact de la surface de la dent avec une composition de blanchiment des dents pendant une durée suffisante pour effectuer un blanchiment de la surface de la dent ;
dans lequel l'au moins un activateur de blanchiment comprend du nonanoyloxybenzènesulfonate de sodium.

**5.** Procédé selon la revendication 4, dans lequel dans la composition de blanchiment des dents, l'au moins un activateur de blanchiment est présent en une quantité supérieure à une quantité de l'au moins un agent de blanchiment.

**6.** Procédé selon la revendication 4 ou la revendication 5, dans lequel dans la composition de blanchiment des dents, l'au moins un agent de blanchiment est présent en une quantité supérieure à environ 0 % en poids et inférieure à environ 10 %.

**7.** Procédé selon l'une quelconque des revendications 4 à 6, dans lequel la composition de blanchiment des dents comprend un composant aqueux.

**8.** Procédé selon l'une quelconque des revendications 4 à 7, dans lequel la composition de blanchiment des dents comprend un gel.

**9.** Procédé selon l'une quelconque des revendications 4 à 8, dans lequel la première chambre de stockage et la seconde chambre de stockage sont des chambres de stockage d'un distributeur de produit de soins bucco-dentaires.

**10.** Procédé selon l'une quelconque des revendications 4 à 9, dans lequel la première chambre de stockage et la seconde chambre de stockage comprennent une partie d'un distributeur de produit de soins bucco-dentaires, dans lequel le distributeur de produit de soins bucco-dentaires comprend en outre une chambre de mélange reliée à la fois à la première chambre de stockage et à la seconde chambre de stockage, et dans lequel la combinaison comprend le mélange au moins partiel de l'au moins un agent de blanchiment et de l'au moins un activateur de blanchiment dans la chambre de mélange.

**11.** Procédé selon l'une quelconque des revendications 4 à 10, dans lequel la combinaison comprend le mélange de l'au moins un agent de blanchiment et de l'au moins un activateur de blanchiment sur une surface d'une gouttière.

**12.** Procédé selon l'une quelconque des revendications 4 à 11, comprenant en outre le remplissage au moins partiel de la première chambre, de la seconde chambre ou à la fois des première et seconde chambres avec au moins un des agents aromatisants, des édulcorants, des agents désensibilisants, des agents anti-microbiens, des agents anti-caries, des agents antitartre, des agents anti-inflammatoires, des vitamines, des pigments et des agents colorants, et des conservateurs.

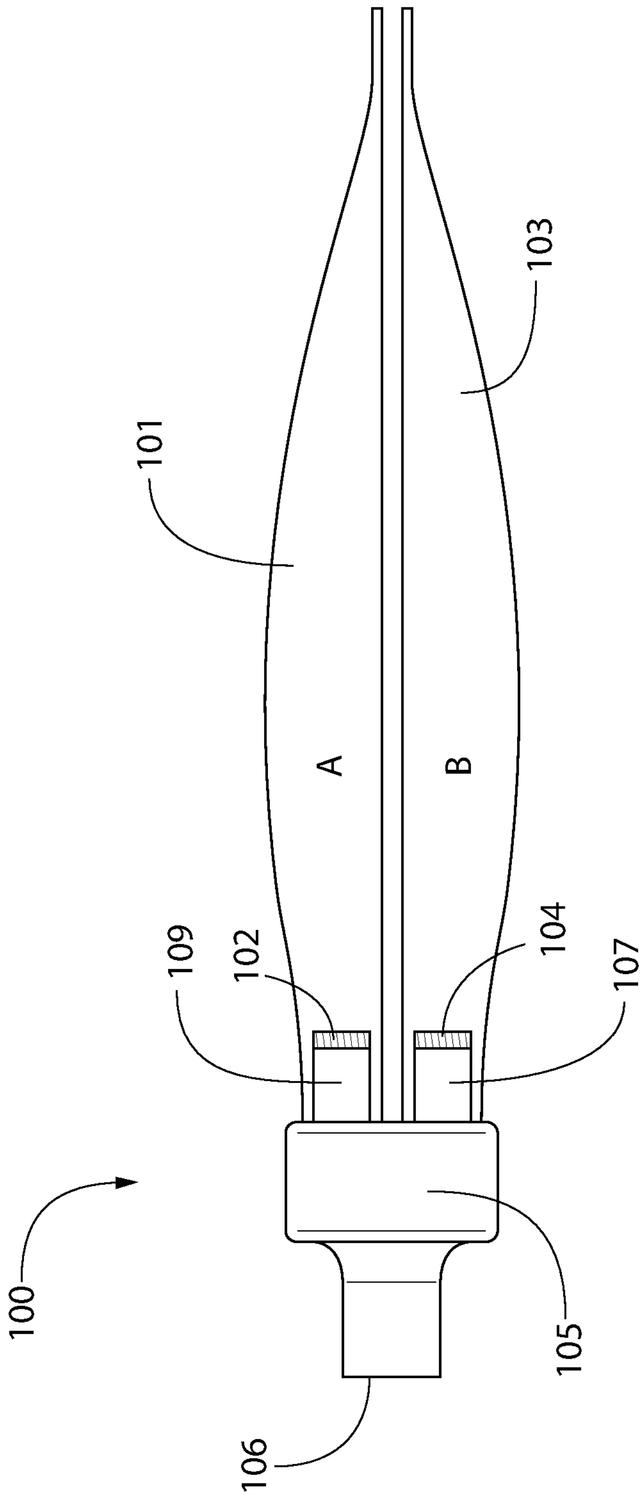


FIG. 1

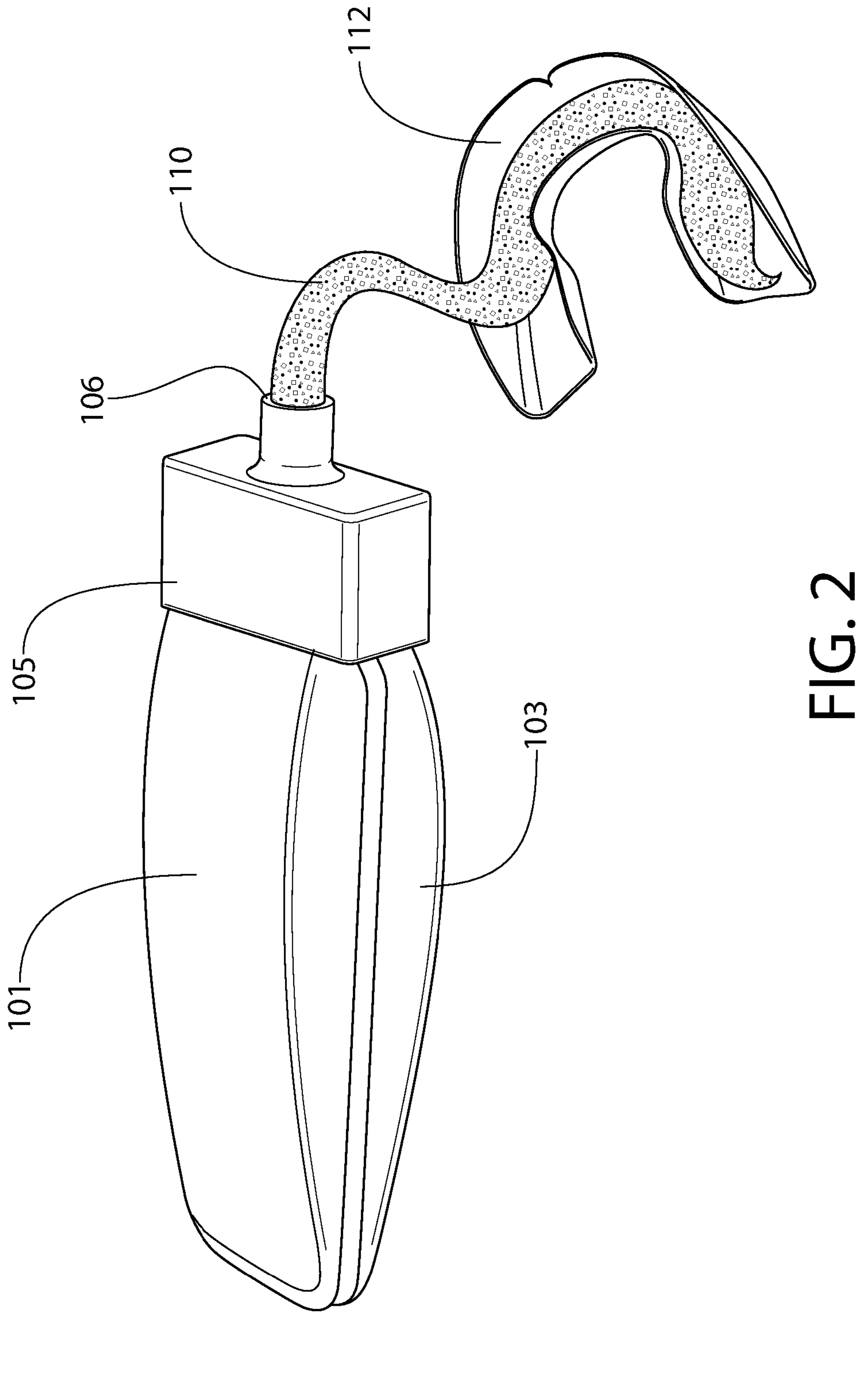


FIG. 2

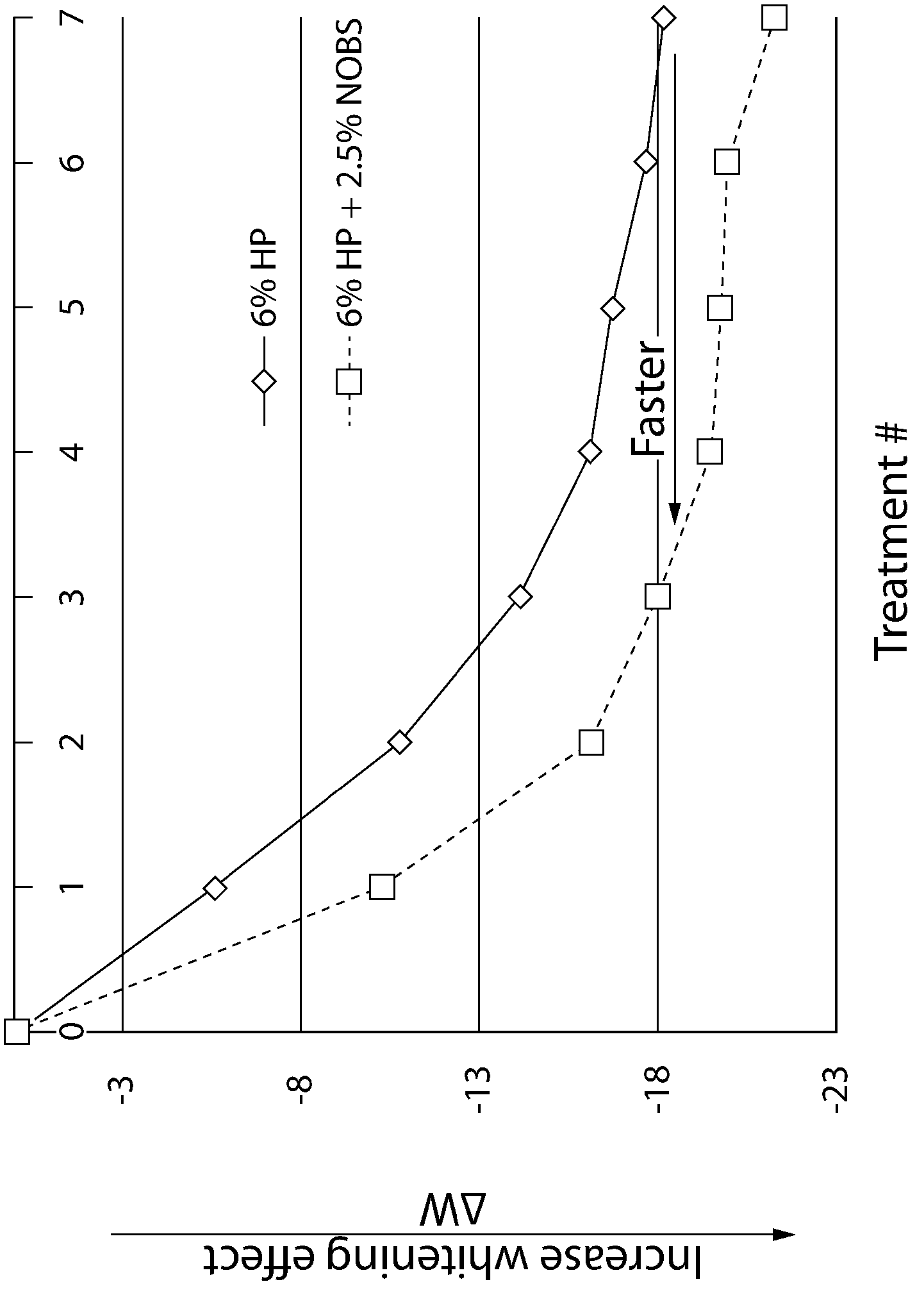

6% HP
6% HP + 2.5% NOBS
Faster
Treatment #
0
1
2
3
4
5
6
7
-3
-8
-13
-18
-23
ΔW
Increase whitening effect


FIG. 3

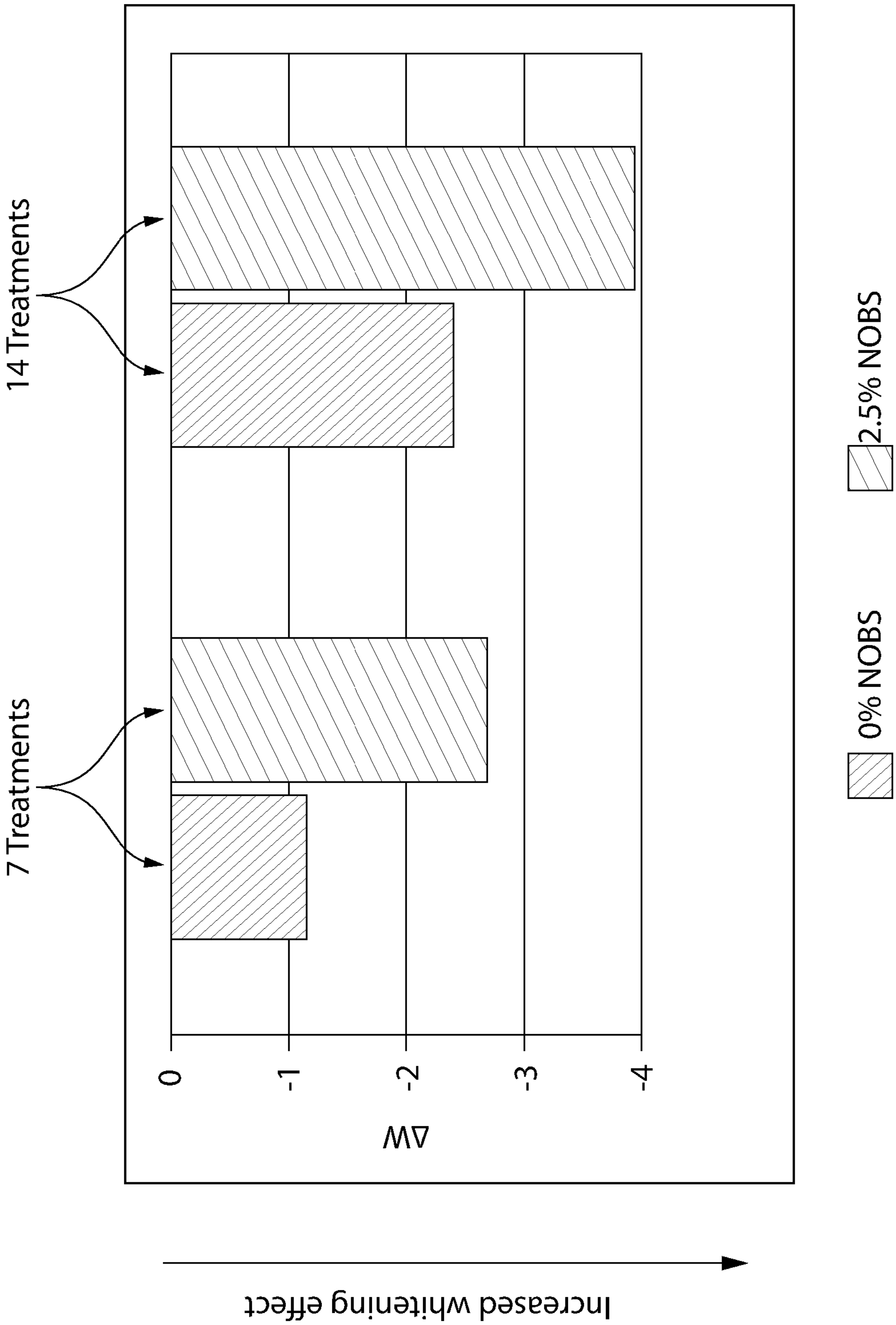
7 Treatments
14 Treatments
0
-1
-2
-3
-4
ΔW
Increased whitening effect
0% NOBS
2.5% NOBS

FIG. 4

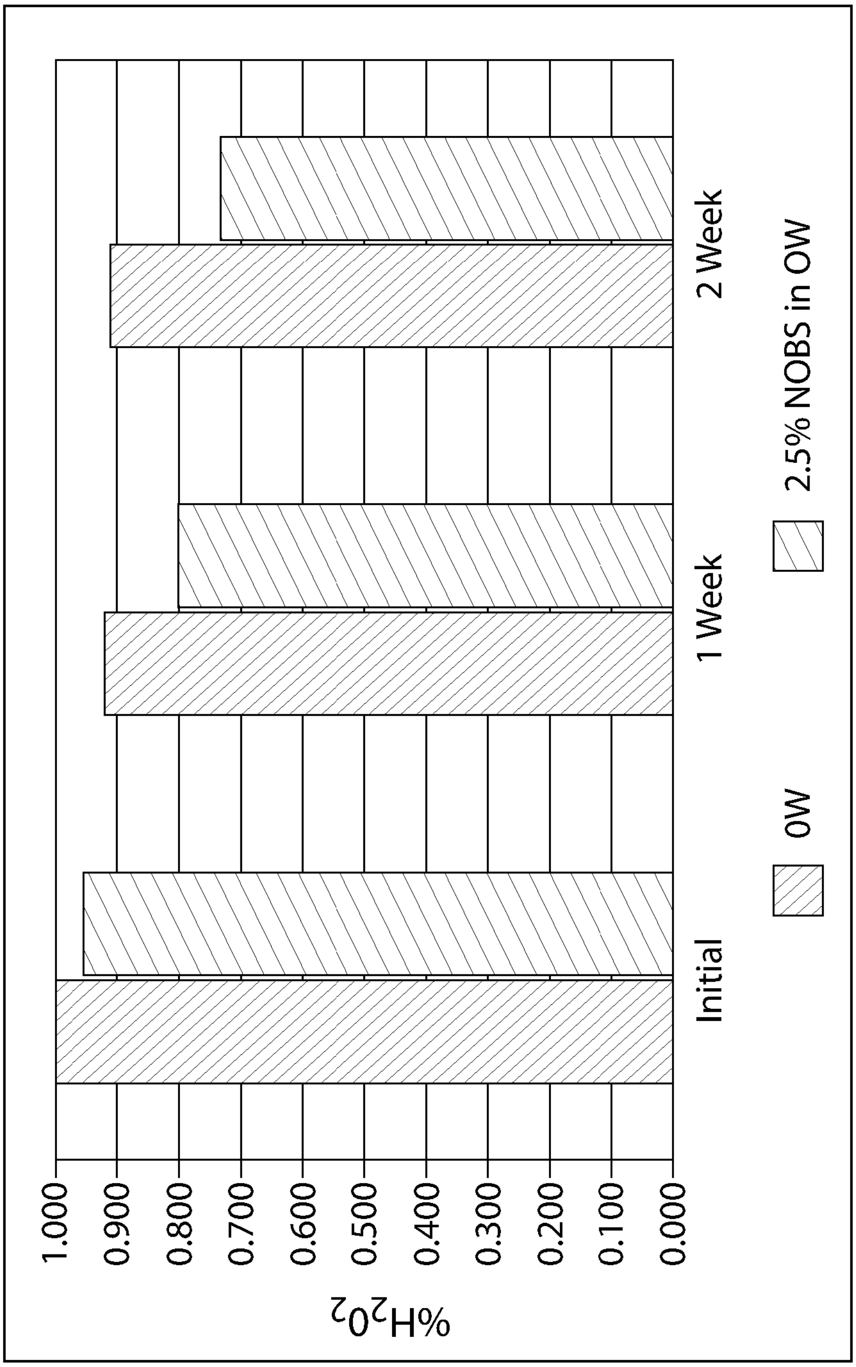
%$H_2O_2$
1.000
0.900
0.800
0.700
0.600
0.500
0.400
0.300
0.200
0.100
0.000
Initial
1 Week
2 Week
OW
2.5% NOBS in OW

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KARST et al.** *Anal. Chem.,* 1997, vol. 69, 3623-3627